(19) Europäisches Patentamt
European Patent Office
Office européen des brevets

(11) Publication number: **0 348 494 B1**

(12) **EUROPEAN PATENT SPECIFICATION**

(45) Date of publication of patent specification: **10.08.94**

(51) Int. Cl.5: **C07D 321/00**, C07D 493/10, C07F 9/12, C07D 311/22, C07D 311/58, C07C 49/83, C07C 49/84, C07C 69/753, C07C 69/017, C07C 69/157, G01N 33/573

(21) Application number: **89901898.0**

(22) Date of filing: **03.01.89**

(86) International application number: **PCT/US89/00015**

(87) International publication number: **WO 89/06650 (27.07.89 89/16)**

# (54) DIOXETANES FOR USE IN ASSAYS.

(30) Priority: **31.12.87 US 140035**

(43) Date of publication of application: **03.01.90 Bulletin 90/01**

(45) Publication of the grant of the patent: **10.08.94 Bulletin 94/32**

(84) Designated Contracting States: **DE FR GB IT**

(56) References cited:
**US-A- 3 720 622**
**US-A- 4 663 278**

**Zaklika "Mechanisms ...", Photochem. Photobiol., 30, 35 (1979).**

**Zaklika "Substituent ...", J. Amer. Chem. Soc., 100, 4916 (1978)**

**Schaap "Chemiluminescence ...", J. Amer. Chem. Soc., 104, 3504 (1982).**

(73) Proprietor: **TROPIX, INC.**
**47 Wiggins Avenue**
**Bedford, MA 01730(US)**

(72) Inventor: **BRONSTEIN, Irena, Y.**
**11 Ivanhoe Street**
**Newton, MA 02153(US)**
Inventor: **EDWARDS, Brooks**
**28 Inman Street**
**Cambridge, MA 02139(US)**
Inventor: **VOYTA, John, C.**
**20 Williams Road**
**North Reading, MA 01864(US)**
Inventor: **KRICKA, Larry, J.**
**886 Nathan Hale Road**
**Berwyn, PA 19312(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 348 494 B1

Schaap "Substituent Effects ...", Tetrahedron Lett., 2943 (1982).

Adam, etal., "Thermal Stability ...", Chem. Ber., 116, 839 (1983).

TETRAHEDRON LETTERS, vol. 28, No. 11, March 1987, pages 1159-1162, Pergamon Journals Ltd., Oxford, GB; A.P. SCHAAP et al.: "Chemical and enzymatic triggering of 1,2-dioxetanes. 3: alkanine phosphatase-catalyzed chemiluminescence from an aryl phosphate-substituted dioxetane".

(74) Representative: **Goldin, Douglas Michael et al**
**J.A. KEMP & CO.**
**14, South Square**
**Gray's Inn**
**London WC1R 5LX (GB)**

## Description

### Background of the Invention

This invention relates to using dioxetanes to detect a substance in a sample.

Dioxetanes are compounds having a 4-membered ring in which 2 of the members are oxygen atoms bonded to each other. Dioxetanes can be thermally or photochemically decomposed to form carbonyl products, e.g., ketones or aldehydes. Release of energy in the form of light (i.e. luminescence) accompanies the decompositions.

Tetrahedron Letters, Vol. 28, No. 11, pages 1156-1162, 1987 describes the preparation of a 9H-xanthen phosphate-substituted 1,2-dioxetane and the enzymatic cleavage of the dioxetane, which is described as chemiluminescent.

### Summary of the Invention

In general, the invention features a dioxetane having the formula

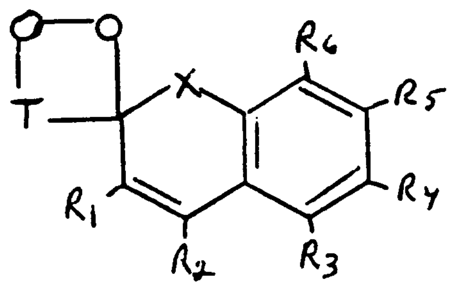

(1)

where T is a substituted (e.g., containing one or more $C_1$-$C_7$ alkyl groups, heteroatom containing groups, e.g., carbonyl groups or enzyme cleavable groups, i.e. groups having a bond that can be cleaved by an enzyme to yield an electron-rich moiety bonded to the dioxetane, e.g., phosphate) or unsubstituted aryl (having between 6 and 12 carbon atoms, inclusive, in the ring, e.g., phenyl), polyaryl (having 2 or more rings, e.g., naphthyl), cycloalkylidene (having between 6 and 12 carbon atoms, inclusive, in the ring) or a polycycloalkylidene (having 2 or more fused rings, each ring independently having between 5 and 12 carbon atoms, inclusive) spiro-bound group at the 3-carbon of the dioxetane; X is $CR_7R_8$, O, S, or N-R (where each $R_7$, $R_8$, and R, independently, is H; a branched or straight chain alkyl group having between 1 and 20 carbon atoms, inclusive, e.g., methyl; a branched or straight chain heteroalkyl having between 1 and 7 carbon atoms, inclusive, e.g., methoxy, hydroxyethyl, or hydroxypropyl; aryl having 1 or 2 rings, e.g., phenyl; heteroaryl having 1 or 2 rings, e.g., pyrrolyl or pyrazolyl; cycloalkyl having between 3 and 7 carbon atoms, inclusive, in the ring. e.g., dioxane; aralkyl having 1 or 2 rings, e.g., benzyl; alkaryl having 1 or 2 rings, e.g., tolyl; or an enzyme cleavable group as defined above); and each $R_1$, $R_2$, $R_3$, $R_4$, $R_5$, and $R_6$ independently, can be H; an electron-withdrawing group (e.g., perfluoroalkyl having between 1 and 7 carbon atoms, inclusive, e.g., trifluoromethyl; halogen; $CO_2H$, $ZCO_2H$, $SO_3H$, $ZSO_3H$, $NO_2$, $ZNO_2$, C = N, or ZC = N, where Z is a branched or straight chain alkyl group having between 1 and 7 carbon atoms, inclusive, e.g., methyl or an aryl group having 1 or 2 rings, e.g., phenyl); an electron-donating group (e.g., a branched or straight chain $C_1$-$C_7$ alkoxy, e.g., methoxy or ethoxy; aralkoxy having 1 or 2 rings, e.g., phenoxy; a branched or straight chain $C_1$-$C_7$ hydroxyalkyl, e.g., hydroxymethyl or hydorxyethyl; hydroxyaryl having 1 or 2 rings; e.g., hydroxyphenyl; a branched or straight chain $C_1$-$C_7$ alkyl ester, e.g., acetate; or aryl ester having 1 or 2 rings, e.g., benzoate); heteroaryl having 1 or 2 rings, e.g., benzoxazole, benzthiazole, benzimidazole, or benztriazole; or an enzyme cleavable group as defined above. Where group T is an aryl or polyaryl group, obviously that aryl group must be one whose structure permits spiro linkage, and thus groups, e.g., phenyl, which cannot be spiro-linked, are excluded.

In addition, as will become apparent from the text which follows, where triggering of the dioxetane is to be by cleavage of the 0-0 bond of the dioxetane ring, neither group T nor the luminescent moiety containing groups $R_1$-$R_6$ need contain an enzyme-cleavable group. Where triggering is by cleavage of an enzyme-cleavable group, either T or the luminescent moiety must contain such a group.

The dioxetane is capable of decomposing to form a luminescent substance (i.e. a substance that emits energy in the form of light) having the formula

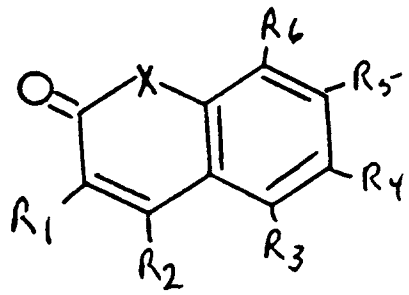

(2)

The invention also features various compounds useful as intermediates in synthesizing the dioxetanes.

In preferred embodiments, group X is O, groups $R_1$-$R_3$ and $R_6$ are H, and groups $R_4$ and $R_5$, independently, are phosphate or H. Group T of the dioxetane is preferably an adamantylidene group. The dioxetane also preferably includes an enzyme cleavable group which may be bonded to group T or to the fluorescent chromophore portion of the dioxetane (preferably at $R_5$).

The dioxetanes are used in an assay method in which a member of a specific binding pair (i.e. two substances that bind specifically to each other) is detected by means of an optically detectable reaction. According to this method, the dioxetane is contacted with an enzyme that causes the dioxetane to decompose to form a luminescent substance (i.e. a substance that emits energy in the form of light). The luminescent substance, which has the formula

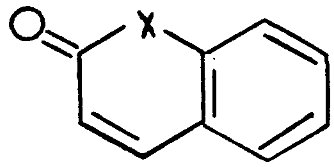

(3)

is detected as an indication of the presence of the first substance. By measuring the intensity of luminescence, the concentration of the first substance can be determined.

Where the enzyme is an oxido-reductase (preferably a peroxidase, e.g., horseradish peroxidase or microperoxidase), it causes the dioxetane to decompose by cleaving the 0-0 bond of the 4-membered ring portion of the dioxetane. The enzyme can act directly on the dioxetane substrate or can be mediated through the addition of peroxide.

Where the dioxetane includes an enzyme cleavable group (e.g., phosphate), the enzyme (e.g., phosphatase) causes the dioxetane to decompose by cleaving the enzyme cleavable group from the dioxetane. Cleavage yields a negatively charged atom (e.g., an oxygen atom) bonded to the dioxetane, which in turn destabilizes the dioxetane, causing it to decompose and emit radiation, which in turn is absorbed by the portion of the molecule containing the fluorescent chromophore, which consequently luminesces.

Preferred specific binding pairs for which the above-described assay method is useful include an antigen-antibody pair, and a pair consisting of a nucleic acid and a probe capable of binding to all or a portion of the nucleic acid. The dioxetanes are also useful in an assay method for detecting an enzyme in a sample.

The invention provides a simple, very sensitive method for detecting substances in samples, e.g., biological samples, and is particularly useful for substances present in low concentrations. Because dioxetane decomposition serves as the excitation energy source for the coumarin chromophore, an external excitation energy source, e.g., light, is not necessary.

Enzyme-triggered decomposition allows for high sensitivity because one enzyme molecule can cause many dioxetane molecules to luminesce, thus creating an amplification effect. In addition, through appropriate modifications of the T group and fluorescent chromophore, the solubility of the dioxetane and the Kinetics of dioxetane decomposition can be varied. The dioxetanes can also be attached to a variety of molecules, e.g., proteins or haptens, or immobilization substrates, e.g., polymer membranes, or included as a side group in a homopolymer or copolymer.

The spiro linkage joining the chromophore to the 4-membered ring portion of the dioxetane stabilizes the dioxetane prior to enzyme activation, making it easy to store the dioxetane for extended periods of time. By using coumarin as the fluorescent chromophore, good quantum yields of luminescence are obtained (as used herein, "quantum yield" refers to the number of photons emitted from the luminescent product per number of moles of dioxetane decomposed). The coumarin-substituted dioxetane molecules are also readily synthesized in good yield.

A further advantage is that where the enzyme acts directly on the 0-0 bond, engineering of the emitting chromophore is not necessary.

Other features and advantages of the invention will be apparent from the following description of the preferred embodiments thereof, and from the claims.

Description of the Preferred Embodiments

We now describe the structure, synthesis, and use of preferred embodiments of the invention.

Structure

The invention employs dioxetanes having the formula

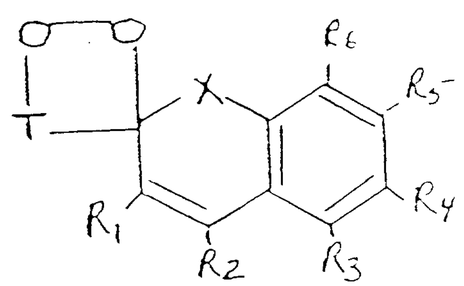

(1)

The purpose of group T is to stabilize the dioxetane, i.e. to prevent premature decomposition. Large, bulky, sterically hindered molecules, e.g., fused polycyclic molecules, are the most effective stabilizers. In addition, T preferably contains only C-C and C-H single bonds. The most preferred molecule is an adamantylidene group consisting of 3 fused cyclohexyl rings. The adamantylidene group is spiro-bound at the 3-carbon of the dioxetane.

The group of formula

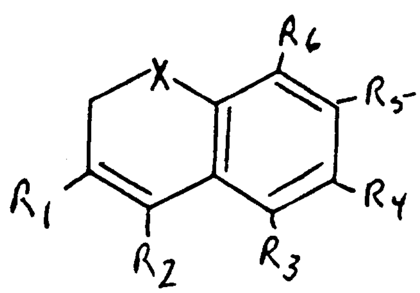

(herein referred to as group V) is a fluorescent chromophore which is spiro-bound at the 4-carbon of the dioxetane. It becomes luminescent when enzymatic cleavage, either of an enzyme cleavable group bonded to group T or group V, or of the 0-0 bond of the 4-membered dioxetane ring, occurs to cause

decomposition of the dioxetane. The decomposition produces two individual carbonyl-containing compounds, one of which contains group T, and the other of which contains group V; the energy released from dioxetane decomposition causes the chromophore to luminesce. Preferably, the excited state energy of group V (i.e. the energy it must possess in order to emit light) is less than the excited state energy of the ketone containing group T in order to confine luminescence to group V.

The preferred dioxetane has the formula

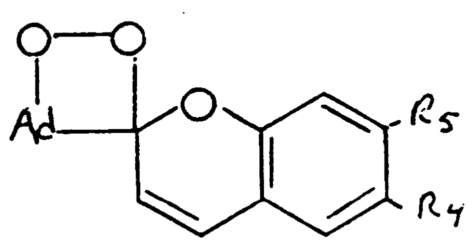

where $R_4$ and $R_5$ independently, are H or phosphate and Ad is an adamantylidene group. Decomposition yields a coumarin molecule having the formula

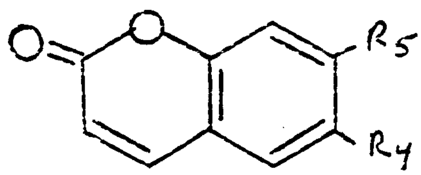

whose excited state energy is less than that of spiroadamantanone (the other decomposition product).

The dioxetanes are generally decomposed in two ways. One way is to add an oxido-reductase enzyme, e.g., a peroxidase (preferably horseradish or microperoxidase); the enzyme cleaves the 0-0 bond of the 4-membered ring, thereby generating the luminescent molecule.

A second way is to bond an enzyme cleavable group to group T or, more preferably, to group V. Contact with the appropriate enzyme cleaves the enzyme-cleavable bond, yielding an electron-rich moiety bonded to group V or group T; this moiety initiates the decomposition of the dioxetane into 2 individual carbonyl-containing compounds. Examples of electron-rich moieties include oxygen, sulfur, and amine or amido anions. The most preferred moiety is an oxygen anion. Examples of suitable enzyme-cleavable groups, and the enzymes specific to these groups, are given below in Table 1; an arrow denotes the enzyme-cleavable bond. The most preferred group is a phosphate ester, which is cleaved by alkaline or acid phosphatase enzymes.

## Table 1

| Group Z | Enzyme |
|---|---|
| 1) 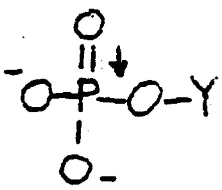 phosphate ester | alkaline and acid phosphatases |
| 2) 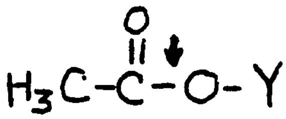 acetate ester | esterases |
| 3) 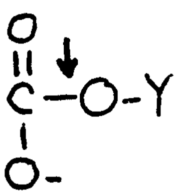 carboxyl | decarboxylases |
| 4) 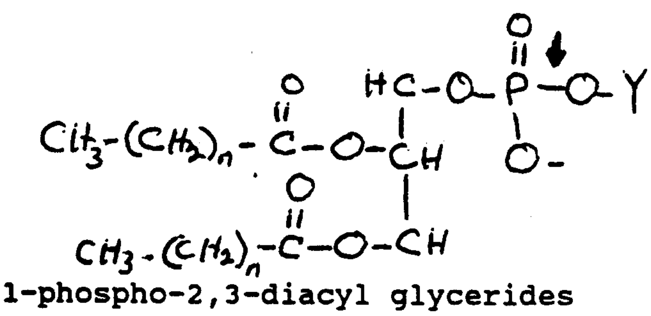 1-phospho-2,3-diacyl glycerides | phospholipase D |

5) 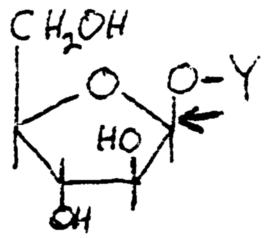 β-xylosidase

β-D-xyloside

6) 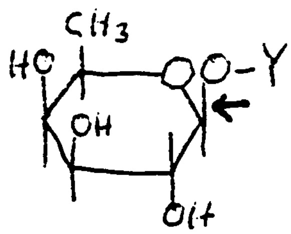 β-D-fucosidase

β-D-fucoside

7) 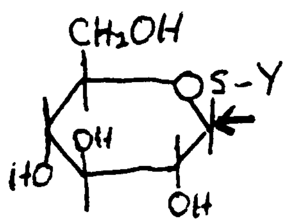 thioglucosidase

1-thio-D-glucoside

8) 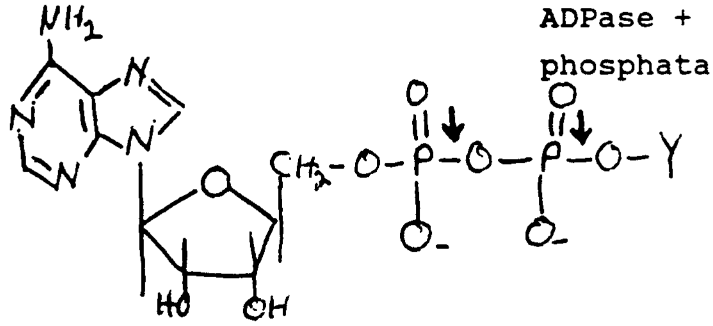 ADPase + phosphatase

adenosine diphosphate analogs

9) 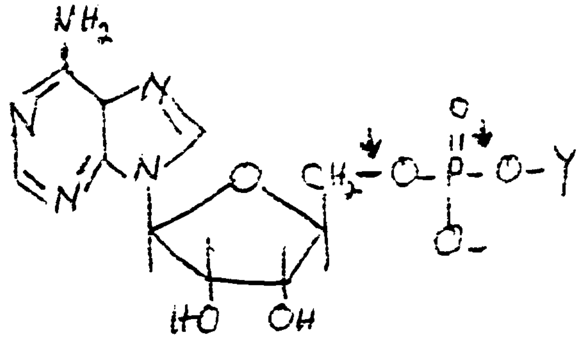 5'-nucleotidase + phosphatase

adenosine monophosphate analogs

10) 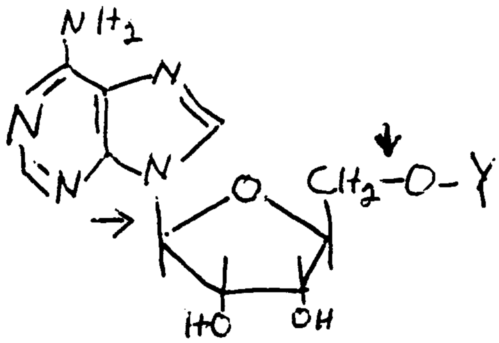 5'-nucleosidase + ribofuranosidase

adenosine analogs

11) 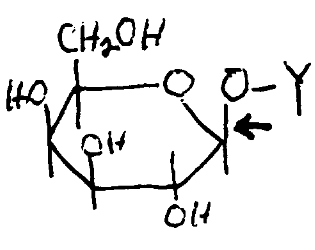 β-D-galactosidase

β-D-galactoside

12) 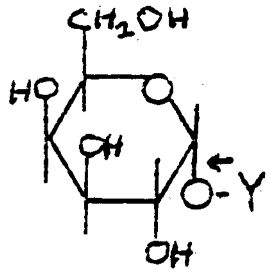 α-D-galactosidase

α-D-galactoside

13) 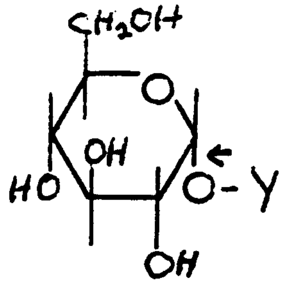 α-D-glucosidase

α-D-glucoside

14) 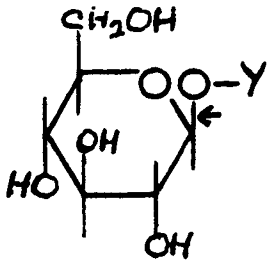 β-D-glucosidase

β-D-glucoside

15) 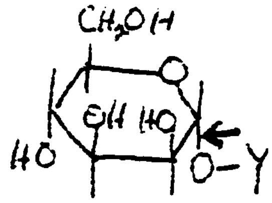 α-D-mannosidase

α-D-mannoside

16) 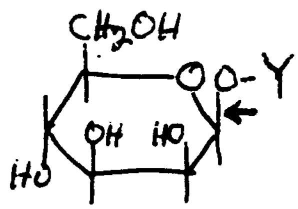 β-D-mannosidase

β-D-mannoside

17) 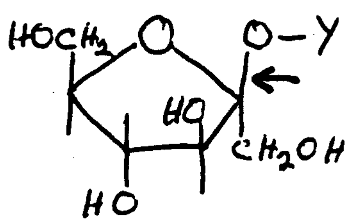 β-D-fructofuranosidase

β-D-fructofuranoside

18) 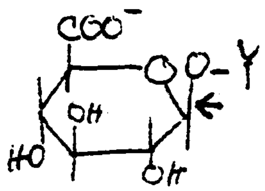 β-D-glucosiduronase

β-D-gludosiduronate

19) 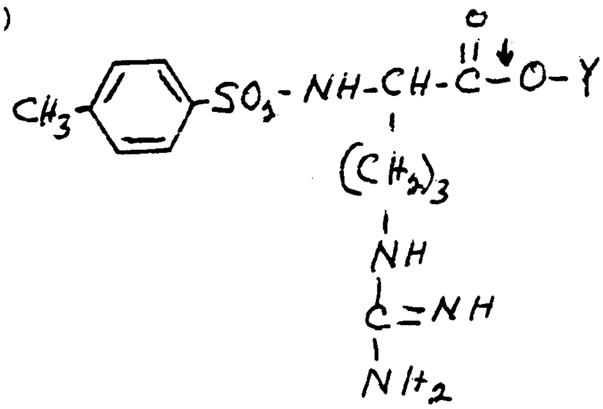 trypsin

p-toluenesulfonyl-L-arginine ester

20) 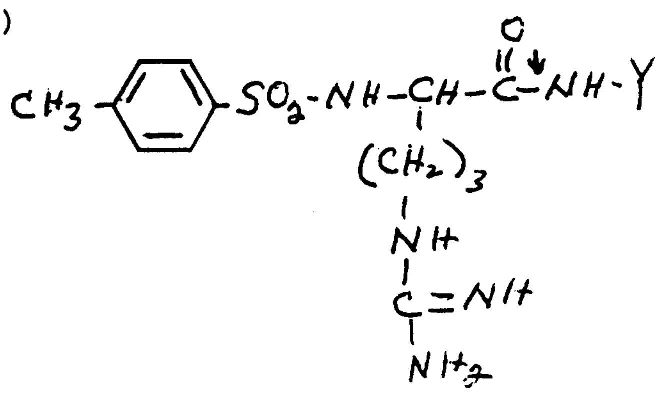 trypsin

p-toluenesulfonyl-L-arginine amide

21) 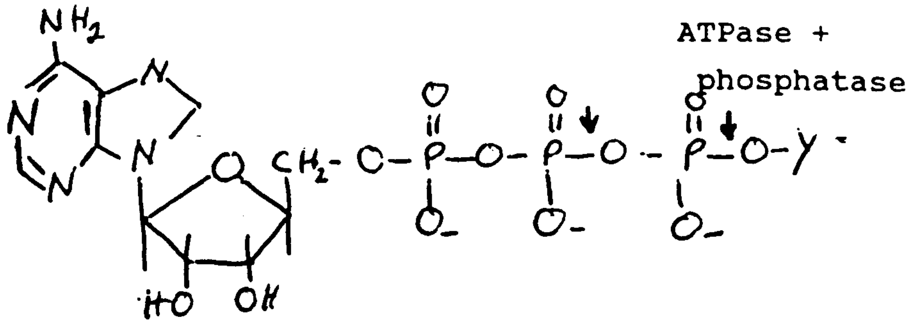


**adenosine triphosphate analogs**

Preferably, the enzyme is covalently bonded to a substance having a specific affinity for the substance being detected. Examples of specific affinity substances include antibodies, e.g., anti-hCG, where the substance being detected is an antigen, e.g., hCG; antigens, e.g., hCG, where the substance being detected is an antibody, e.g., anti-hCG;or a probe capable of binding to all or a portion of a nucleic acid, e.g. DNA or RNA, being detected. Bonding is preferably through an amide bond.

Synthesis

In general, the dioxetanes of the invention are synthesized in two steps. The first step involves synthesizing an appropriately substituted olefin having the formula

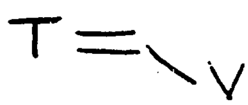

where T and V are as described above in which chromophore V is spiro bound at the olefin double bond are generally synthesized using one of the following two synthetic methods.

The first synthesis involves using the following reaction sequence:

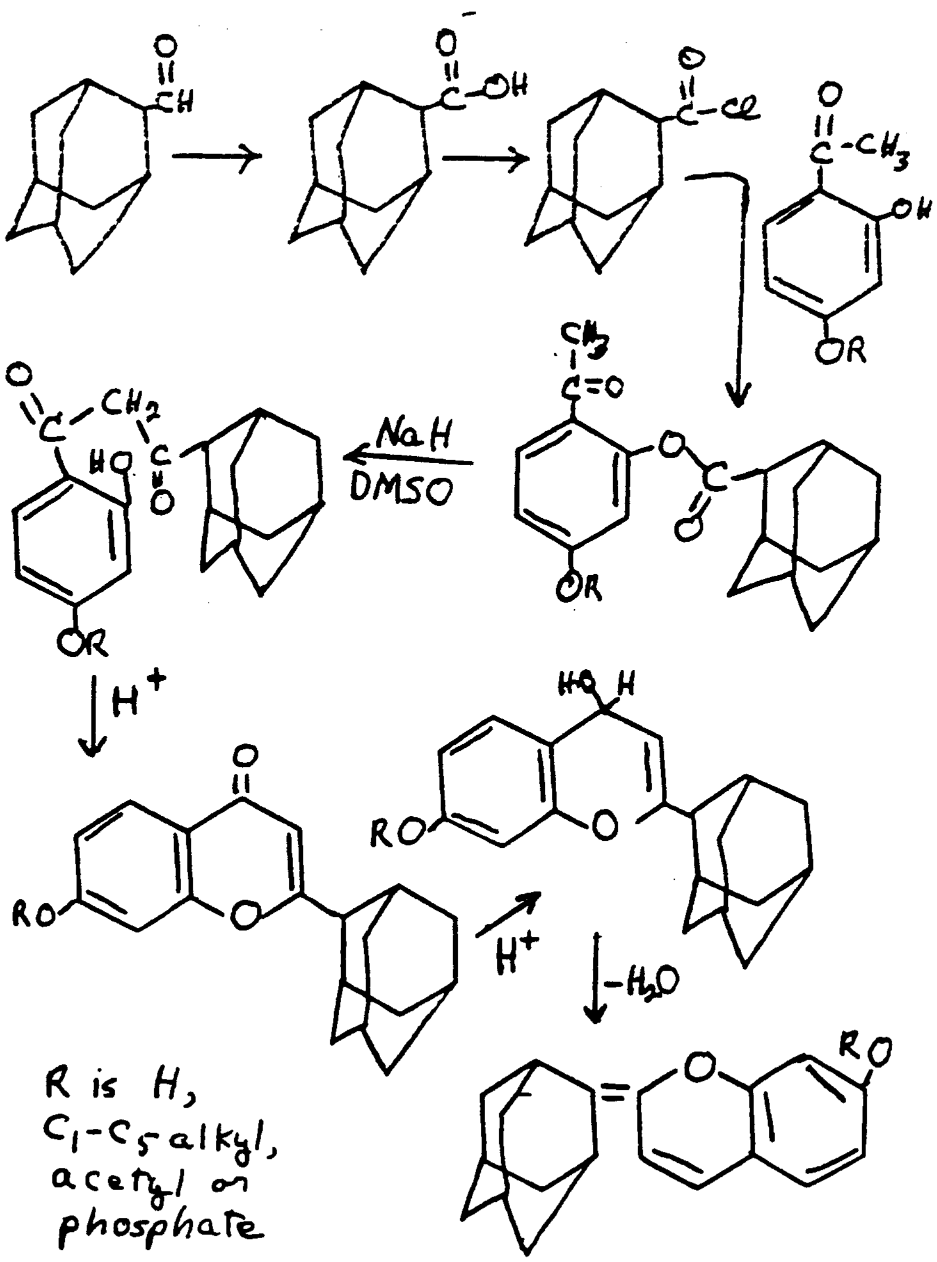


The reaction was carried out as follows for the case where R is methyl.

Adamantane-2-carboxylic acid (1.51g., 8.38 mmol), prepared following the procedure of Farcasiu (Synthesis, 1972, 615), was dissolved in 35mL $CH_2Cl_2$. The solution was cooled in an ice bath to produce a thin suspension. Oxalyl chloride (.88mL, 10 mmol) was added with stirring. The subsequent addition of 2 drops DMF produced immediate evolution of gas. After 15 minutes the mixture was warmed to room temperature and stirring was continued for 2 hours. The volatiles were then removed under reduced pressure. The crude acid chloride was diluted with 25mL sieve dried (3Å) pyridine to provide a slightly cloudy, yellow solution upon cooling an ice bath.

2'-hydroxy-4'-methyoxyacetophenone (1.18g., 7.12 mmole) in 5mL pyridine was added to the solution dropwise. The mixture was stirred at 0 °C for 30 minutes and then warmed to room temperature for an additional 2 hours. The mixture was poured into saturated $NaHCO_3$ solution (150mL) and extracted with (3x20mL) 25% ethyl acetate in hexanes. The combined organics were washed with 1N HCl, saturated $NaHCO_3$, and water, followed by drying over $Na_2SO_4$. The solution was concentrated to provide 2.5g. of a yellow oil. I.R. (film) :2900 $cm^{-1}$ (ester C = 0), 1672 $cm^{-1}$ (ketone C = 0). TLC analysis showed that the ester product, 2'-(Adamantane-2-carbonyloxy)-4'-methoxyacetophenone, was sufficiently pure for subsequent use.

The ester obtained above (1.53g., 4.6 mmol) was next dissolved in 10mL DMSO, and added dropwise to a suspension of NaH (60% dispersion - .56g, 13.97 mmole) in 35mL DMSO. After foaming had ceased, the brown mixture was stirred for 30 minutes at room temperature. The mixture was poured into saturated oxalic acid solution, and extracted with 3x50mL ethyl acetate. The combined organics were washed several times with water and finally with saturated NaCl. Concentration and chromatography through a short silica gel plug using 10% ethyl acetate in hexanes provided 1.58g. of a slightly orange solid. I.R. ($CHCl_3$): 2900cm$^{-1}$, 1695cm$^{-1}$ (C=0). An analytical sample of the product 1,3-diketone; 2-(Adamantane-2-carbonyl)-2'-hydroxy-4'-methoxyacetophenone, recrystallized from hexanes exhibited a melting point of 105-108 °C.

The crude diketone product of the previous reaction (1.58g) was suspended in 40mL acetic acid and treated with 15 drops conc. HCl. The mixture was heated at 100-110 °C for 30 minutes. The mixture was carefully neutralized with $NaHCO_3$ solution and extracted several times with ethyl acetate. The combined organic layers were washed with water and saturated NaCl. The solution was dried quickly over $Na_2SO_4$ and evaporated to yield 1.5g of a slightly brown solid which could be recrystallized from ethyl acetate to give .92 gms. of the chromenone, 2-(Adamant-2-yl)-7-methoxy-4H-chromen-4-one, as light buff crystals. I.R. ($CHCl_3$): 2900 cm$^{-1}$, 1630 cm$^{-1}$ (C=0), 1595 cm$^{-1}$, 1435 cm$^{-1}$, m.p. 160-162 °C.

A solution of the above-prepared chromenone (.62g., 2 mmol) in 10mL absolute methanol, was treated with .4m $CeCl_3.7H_2O$ in methanol (5mL, 2 mmol). $NaBH_4$ (76mg., 2 mmol) was added in small portions with stirring. After 60 minutes at room temperature, 50mL water was added and the mixture extracted with 2X20mL ethyl acetate. The combined organic layers were washed first with water and then with saturated NaCl. The solution was dried over $Na_2SO_4$ and concentrated in vacuo to provide the crude allylic alcohol. The light straw colored oil showed no carbonyl absorption (1630 cm$^{-1}$) due to the starting material in the I.R. spectrum. The oil was dissolved in 25mL $CH_2Cl_2$ under argon and the solution was cooled to -20C °. Triethylamine (.61g, 6 mmole) was added by syringe with stirring. Methanesulfonyl chloride (.25g., 2.2 mmole) was then added dropwise. The mixture was allowed to slowly warm up to room temperature, whereupon stirring was continued overnight. The mixture was extracted several times with water, dried over $Na_2SO_4$, and stripped to remove solvent and excess triethylamine. The product, 7-methoxy-2-adamantylidene-3-chromene, was obtained with a yield of .5g. To prepare the phosphorylated version, the chrome is demethylated with sodium ethanethiolate in DMF, and then phosphorylated with 2-chloro-2-oxo-1,3-dioxaphospholane.

The second synthesis involves using the Barton reaction according to the following reaction sequence:

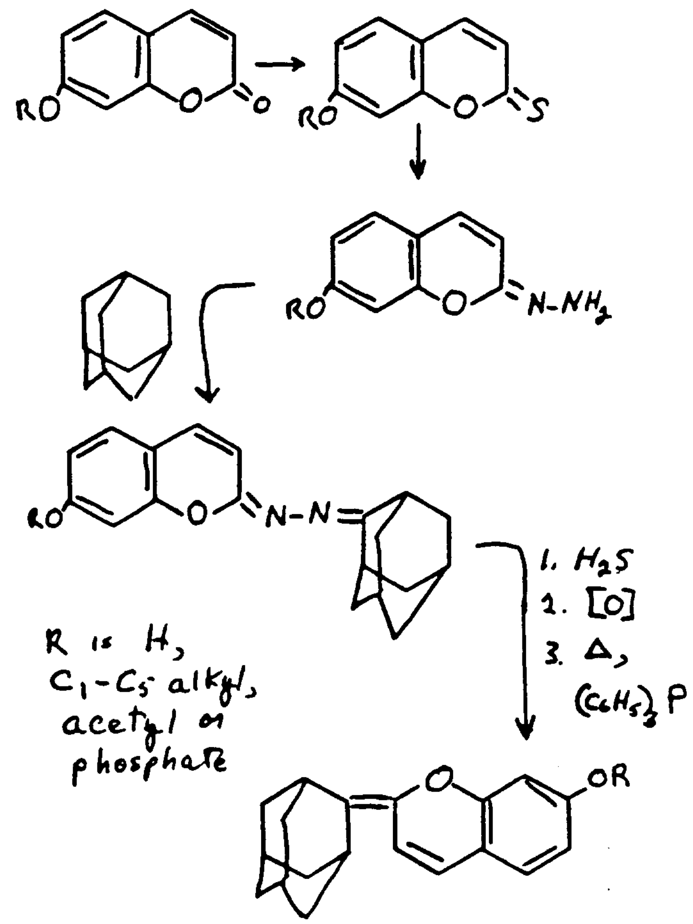


The reaction was carried out as follows for the case where R is methyl.

7-methoxy-coumarin-2-thione (2g, 10.4 mmol), obtained by the procedure of F. Tiemann (Ber. 19, 1661, 1886), and hydrazine monohydrate (.55mL, 11.3 mmol) were heated under reflux in absolute ethanol for 4 hours. The mixture was filtered while hot and the filtrate evaporated under reduced pressure. The residue was extracted several times with boiling petroleum ether (35-60 °C). The solution was concentrated to dryness and the residue was recrystallized from ethanol to provide the hydrazone, 2-hydrazono-7-methoxy-2H-benzopyran, as a light yellow solid.

A solution of the above hydrazone (2.g., 10.5 mmol) and 2-adamantanone (1.6g, 10.7 mmol) in 50mL absolute ethanol was treated with 0.1mL triethylamine and 0.1mL glacial acetic acid. The mixture was stirred overnight at room temperature and subsequently refluxed for 3 hours. The solvent was removed and the residue was chromatographed on silica gel to obtain the mixed azine, 7-methoxy-2-N-benzo-2H-pyranoadamantylideneazine, as a yellow solid.

The azine (3.2g, 10 mmol) was dissolved in 200mL of 1:1 toluene-dimethoxyethane. This solution was then treated with 2 mg. of p-toluene sulfonic acid. Hydrogen sulfide was slowly bubbled in through a gas dispersion tube with vigorous stirring. The reaction was monitored by TLC, which showed complete consumption of the starting material after 18 hours. The solvent was removed under reduced pressure to yield a residue which was triturated with hexanes and dried in vacuo. The product was chromatographed on silica gel using ethyl acetate-dichloromethane to provide the heterocyclic product, 7-methoxy-2H-benzopyranspiro-2'-(1',3',4'-thiadiazolidine)-5'-spiroadamantane, in good yield.

Next, sieve dried benzene (50mL) was stirred under argon during the addition of $CaCO_3$ (3.5g., 35 mmol). Lead tetracetate (3.5g., 7.9 mmol) was added in small portions to the white suspension, which was then allowed to stir for 30 minutes at room temperature. A solution of the aforementioned thaidiazolidine (2.1g, 6 mmol) in 50mL benzene was added dropwise over a one hour period. The mixture was then allowed to stir overnight. Water (200mL) was added with vigorous stirring. The aqueous layer was extracted with 3X30mL benzene and the combined organics were washed with 2x40mL water, 1x40mL saturated

NaCl, and dried over $Na_2So_4$. The solvent was removed in vacuo to yield 3g of residue which was chromatographed on silica gel with dichloromethane-hexanes to provide the off-white crystalline thiadiazine, 7-methoxy-2H-benzopyranspiro-2'-(1',3',4'-thiadiazine)-5'-spiroadamantane, in good yield.

A mixture of the above thiadiazine (1.77g, 5 mmole) and triphenyl phosphine (3.1g., 11.7 mmole) was heated in a sealed tube under argon at 150 °C for 18 hours. The contents were dissolved in a small amount of methylene chloride and applied to a silica gel column for flash chromatography using 5% $CH_2Cl_2$ - hexanes to yield 7-methoxy-2-adamantylide-3-chromene. The product was identical in all respects to the chromene obtained by the first synthetic route. It could also be phosphorylated as described above.

The second step in the synthesis of the dioxetanes involves converting the olefin described above to the dioxetane. Preferably, the conversion is effected photochemically by treating the olefin with singlet oxygen ($^1O_2$) in the presence of light. $^1O_2$ adds across the double bond to form the dioxetane as follows:

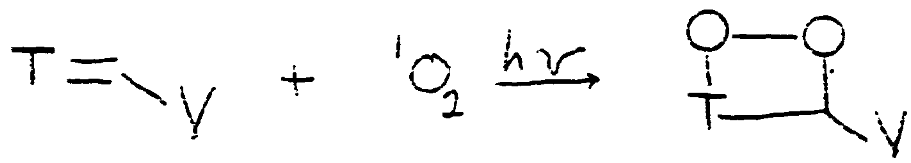

The reaction is preferably carried out a +15 °C in a halogenated solvent, e.g., methylene chloride. $^1O_2$ is generated using a photosensitizer. Examples of photosensitizers include polymer-bound Rose Bengal (commercially known as Sensitox II and available from Polysciences) and methylene blue (a well-known dye and pH indicator). The most preferred sensitizer is methylene blue.

The synthesis of the dioxetane having the formula

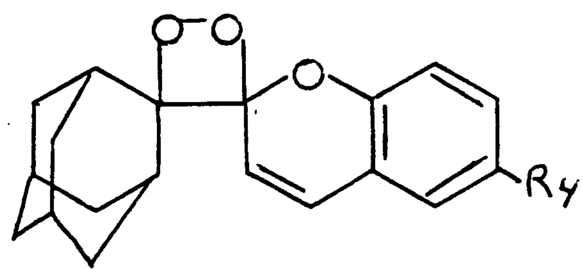

where $R_4$ is a phosphate group follows.

In a large culture tube, .075g (.21 mmole) of the chromene phosphate salt prepared as described above was dissolved in 25mL $CHCl_3$. A quantity (0.210g) of methylene blue on silica gel (0.0026g dye/g $SiO_2$) was added as a sensitizer. The tube was placed in a silvered Dewar flask containing a 250 watt, high-pressure sodium lamp inside a water-cooled immersion well. A piece of 5 mil Kapton (Dupont) placed inside the well served as a U.V. filter. Ice water was pumped through the apparatus to maintain the sample temperature below 15 °C. A continuous stream of dry oxygen was passed into the reaction vessel through a capillary tube. The gas flow was adjusted so as to just maintain a uniform Suspension of solid-phase sensitizer. After 25 minutes irradiation time, the U.V. absorption of the starting material disappeared. The light yellow-green solution was filtered, evaporated, and reconstituted with 10mL water. The agueous sample was then filtered through 0.45 micron nylon filter and chromatographed on a reverse phase, C18 preparative HPLC column suing a water/acetonitrile gradient. The appropriate fractions were combined and lyophilized to provide the dioxetane, dispiro(adamantane-2)-3'-(1',2'-dioxetane)-4',2''-(7''-phosphoryloxy-3''-chromene) sodium salt, as a white, hygroscopic solid.

Use

A wide variety of assays exist which use visually detectable means to determine the presence or concentration of a particular substance in a sample. The above-described dioxetanes can be used in any of these assays. Examples of such assays include immunoassays to detect antibodies or antigens, e.g., α or β-hCG; enzyme assays; chemical assays to detect, e.g., potassium or sodium ions; and nucleic acid assays to detect, e.g., Viruses (e.g., HTLV I or III or cytomegalovirus), or bacteria (e.g., E. coli)).

When the detectable substance is an antibody, antigen, or nucleic acid, the enzyme is preferably bonded to a substance having a specific affinity for the detectable substance (i.e. a substance that binds specifically to the detectable substance), e.g., an antigen, antibody, or nucleic acid probe, respectively. Conventional methods, e.g., carbodiimide coupling, are used to bond the enzyme to the specific affinity substance; bonding is preferably through an amide linkage.

In general, assays are performed as follows. A sample suspected of containing a detectable substance is contacted with a buffered solution containing an enzyme bonded to a substance having a specific affinity for the detectable substance. The resulting solution is incubated to allow the detectable substance to bind to the Specific affinity portion of the specific affinity-enzyme compound. Excess specific affinity-enzyme compound is then washed away, and a dioxetane is added. In the case of an oxido-reductase enzyme, the 0-0 peroxy bond of the dioxetane is cleaved, causing the dioxetane to decompose into 2 ketones, one of which contains the chromophore, e.g., coumarin; the chromophore is thus excited and luminesces. Luminescence is detected using, e.g., a photomultiplier tube detector or camera luminometer, as an indication of the presence of the detectable substance in the sample. Luminescence intensity is measured to determine the concentration of the substance.

Where group T or group V contains an enzyme-cleavable group, e.g., phosphate, the enzyme, e.g., phosphatase, cleaves this group to cause luminescence as described above.

When the detectable substance is an enzyme, a specific affinity substance is not necessary. Instead, a dioxetane is used. Therefore, an assay for the enzyme involves adding the dioxetane to the enzyme-containing sample, and detecting the resulting luminescence as an indication of the presence and the concentration of the enzyme.

Examples of specific assays follow.

A. Assay for Human IgG

A 96-well microtiter plate is coated with sheep anti-human IgG ($F(ab)_2$ fragment Specific). A serum sample containing human IgG is then added to the wells, and the wells are incubated for 1 hour at room temperature. Following the incubation period, the serum sample is removed from the wells, and the wells are washed four times with an aqueous buffer solution containing 0.15M NaCl, 0.01M phosphate, and 0.1% bovine serum albumin (pH 7.4).

Horseradish peroxidase bonded to anti-human IgG is added to each well, and the wells are incubated for 1 hour. The wells are then washed four times with the above buffer solution, and a buffer solution of a dioxetane is added. The resulting luminescence caused by enzymatic degradation of the dioxetane is detected in a luminometer, or with photographic film in a camera luminometer.

Similar results are obtained using a phosphate-containing dioxetane and alkaline phosphatase in place of horseradish peroxidase.

B. Assay for hCG

Rabbit anti-$\alpha$ hCG is adsorbed onto a nylon-mesh membrane. A Sample solution containing HCG, e.g., urine from a pregnant woman, is blotted through the membrane, after which the membrane is washed with 1ml of a buffer solution containing 0.15M NaCl, 0.01M phosphate, and 0.1% bovine serum albumin (pH 7.4).

Microperoxidase-labelled anti-$\beta$-hCG is added to the membrane, and the membrane is washed again with 2ml of the above buffer solution. The membrane is then placed in the cuvette of a luminometer or into a camera luminometer, and contacted with a dioxetane. The luminescence resulting from enzymatic degradation of the dioxetane is then detected.

Similar results are obtained using alkaline phosphatase-labelled anti $\beta$-hCG and a phosphate-containing dioxetane.

C. Assay for Serum Microperoxidase

2.7ml of an aqueous buffer solution containing 0.84M 2-methyl-2-aminopropanol is placed in a 12x75mm pyrex test tube, and 0.1.ml of a serum Sample containing microperoxidase added. The solution is then equilibrated to 30°C. 0.2ml of a dioxetane is added, and the test tube immediately placed in a luminometer to record the resulting luminescence. The level of light emission will be proportional to the rate of microperoxidase activity.

The above-described assay can be used to detect serum alkaline phosphatase by using a phosphate-containing dioxetane.

D. Nucleic Acid Hybridization Assay

A sample of cerebrospinal fluid (CSF) suspected of containing cytomegalovirus is collected and placed on a nitrocellulose membrane. The sample is then chemically treated with urea or quanidinium isothiocyanate to break the cell walls and to degrade all cellular components except the viral DNA. The strands of the viral DNA thus produced are separated and attached to the nitrocellulose filter. A DNA

probe specific to the viral DNA and labelled with horseradish peroxidase is then applied to the filter; the probe hybridizes with the complementary viral DNA strands. After hybridization, the filter is washed with an aqueous buffer solution containing 0.2 M NaCl and 0.1mM Tris-HCl (pH = 8.0) to remove excess probe molecules. A dioxetane is added and the resulting luminescence from the enzymatic degradation of the dioxetane is measured in a luminometer or detected with photographic film.

Similar results are obtained using alkaline phosphatase to label the DNA probe, and a phosphate-containing dioxetane.

Other embodiments are within the following claims.

For example, the specific affinity substance can be bonded to the dioxetane through group T or group V, instead of the enzyme. In this case, the group to which the specific affinity substance is bonded is provided with, e.g., a carboxylic acid, amino, or maleimide substituent to facilitate bonding.

Group T or group V of the dioxetane can be bonded to a polymerizable group, e.g., a vinyl group, which can be polymerized to form a homopolymer or copolymer.

Group T or group V of the dioxetane can be bonded to, e.g., membranes, films, beads, or polymers for use in immuno-or nucleic acid assays. The groups are provided with, e.g., carboxylic acid, amino, or maleimide substituents to facilitate bonding.

Group T or group V of the dioxetane can contain substituents which enhance the kinetics of the dioxetane peroxidase-induced degradation, e.g., electron-rich moieties (e.g., methoxy).

Another example of a chemical synthesis involves converting the olefin precursor to a 1,2-bromohydroperoxide by reacting the olefin with $H_2O_2$ and dibromantin (1,3-dibromo-5,5-dimethyl hydantoin). Treatment of the 1,2-bromohydroperoxide with base, e.g., NaOH, or silver salts, e.g., silver bromide, forms the dioxetane.

Rather than treating the olefin with photochemically generated singlet oxygen, the dioxetane can be prepared by treating the olefin with triphenyl phosphite ozonide or triethylsilyl hydrotrioxide, as described in Posner et al., J. Am. Chem. Soc. 109:278-79 (1987).

Another method of synthesizing the olefin precursor involves the McMurry reaction in which the chromophore and carbonyl form of group T olefin are reacted with $TiCL_3$/LAH to form the olefin. Modified McMurray reactions, e.g., where $TiCl_4$/TMEDA/Zn is used instead of $TiCl_3$/LAH, can also be used.

The invention further provides a kit for detecting a first substance in a sample, the kit comprising

a dioxetane having the formula

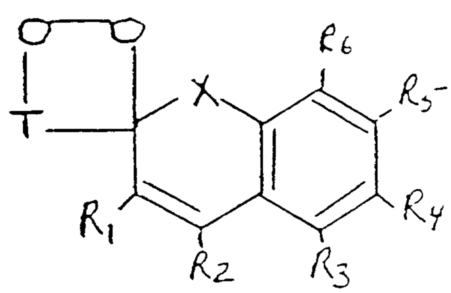

wherein T is a substituted or unsubstituted aryl, polyaryl, cycloalkylidene, or polycycloadlkylidene group spiro-bound at the 3-carbon of said dioxetane, X is $CR_7R_8$, O, S, or N-R (wherein each of $R_7$, $R_8$, and R, independently, is H, alkyl, heteroalkyl, aryl, heteroaryl, cycloalkyl, cycloheteroalkyl, aralkyl, alkaryl, or an enzyme cleavable group), and each of aralkyl, alkaryl, or an enzyme cleavable group), and each of $R_1$, $R_2$, $R_3$, $R_4$, $R_5$, and $R_6$, independently, is H, an electron-withdrawing group, an electron-donating group, heteroaryl, or an enzyme cleavable group; and

an enzyme capable of causing said dioxetane to decompose to form a fluorescent substance having the formula

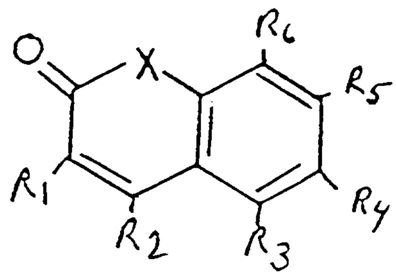

The invention also provides a compound having the formula

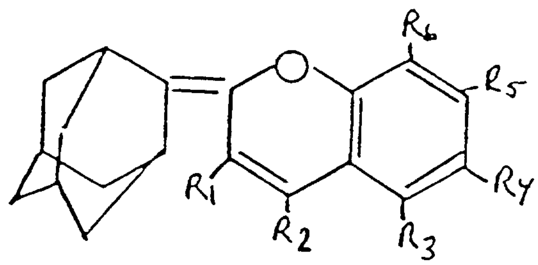

where each of $R_1$, $R_2$, $R_3$, $R_4$, $R_5$, and $R_6$, independently, is H, an electron-withdrawing group, an electron-donating group, hetercaryl, or an enzyme-cleavable group.

**Claims**

**1.** A dioxetane having the formula

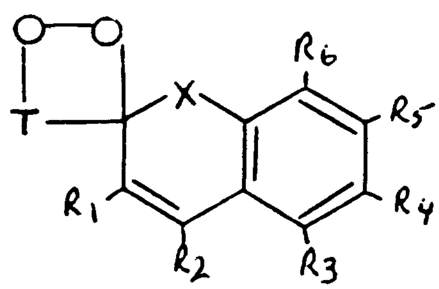

wherein T is a substituted or unsubstituted aryl, polyaryl, cycloalkylidene, or polycycloalkylidene group spiro-bound at the 3-carbon of said dioxetane; X is $CR_7R_8$, O, S, or N-R (wherein each of $R_7$, $R_8$, and R, independently, is H, alkyl, heteroalkyl, aryl, heteroaryl, cycloalkyl, cycloheteroalkyl, aralkyl, alkaryl, or an enzyme cleavable group); and each of $R_1$, $R_2$, $R_3$, $R_4$, $R_5$, and $R_6$, independently is H, an electron-withdrawing group, an electron-donating group, heteroaryl, or an enzyme cleavable group,

said dioxetane being capable of decomposing to form a luminescent substance having the formula

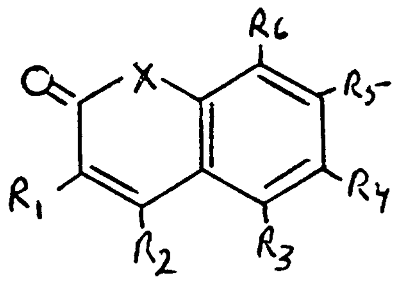

2. A dioxetane according to claim 1 therein at least one of said groups, T, $R_1$, $R_2$, $R_3$, $R_4$, $R_5$, and $R_6$ independently, comprises an enzyme cleavable group.

3. A dioxetane according to claim 2 wherein said enzyme-cleavable group comprises phosphate.

4. A dioxetane according to claim 1 wherein the group X is O, groups $R_1$-$R_3$ and $R_6$ are H, and groups $R_4$ and $R_5$, independently, are H or an enzyme cleavable group.

5. A dioxetane according to any preceding claim wherein the group T is a polycycloalkylidene group.

6. A dioxetane according to claim 5 wherein the group T is adamantylidene.

7. A compound having the formula

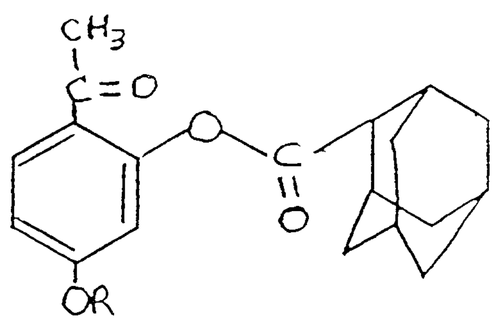

where R is H, $C_1$-$C_5$ alkyl group, acetyl, or $PO_4$.

8. A compound having the formula

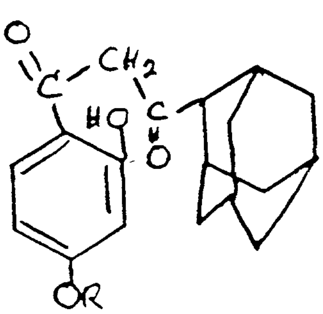

where R is H, a $C_1$-$C_5$ alkyl group, acetyl, or $PO_4$.

**9.** A compound having the formula

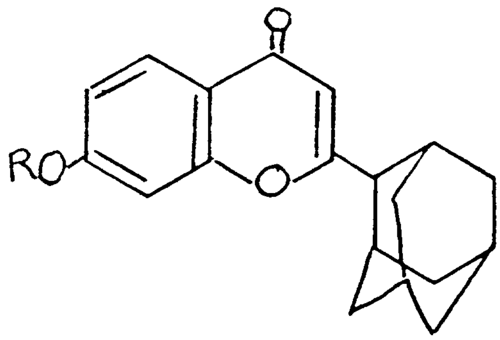

where R is H, a $C_1$-$C_5$ alkyl group, acetyl, or $PO_4$.

**10.** A compound having the formula

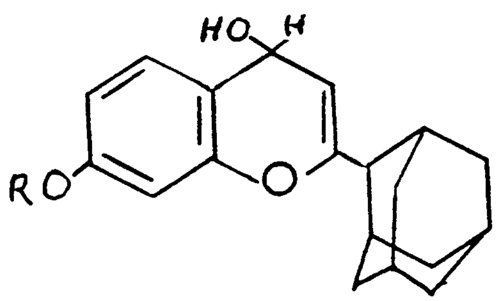

where R is H, a $C_1$-$C_5$ alkyl group, acetyl, or $PO_4$.

**11.** A compound having the formula

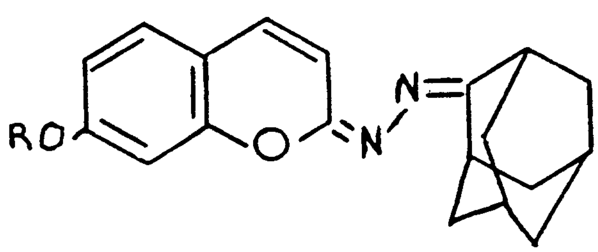

where R is H, a $C_1$-$C_5$ alkyl group, acetyl, or $PO_4$.

**12.** A compound having the formula

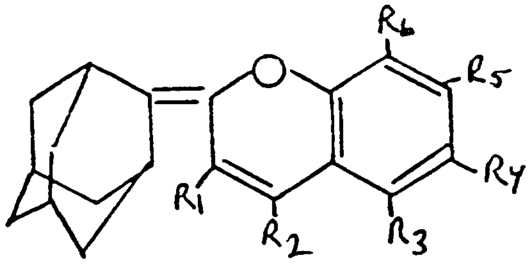

where each of $R_1$, $R_2$, $R_3$, $R_4$, $R_5$, and $R_6$, independently, is H, an electron-withdrawing group, an electron-donating group, heteroaryl, or an enzyme-cleavable group.

**13.** An assay method in which a member of a specific binding pair is detected by means of an optically detectable reaction characterised in that the optically detectable reaction includes the reaction, with an enzyme, of a dioxetane having the formula:

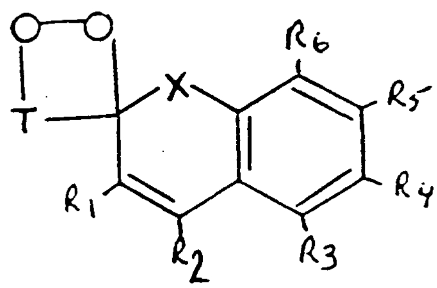

wherein T is a substituted or unsubstituted aryl, polyaryl, cycloalkylidene, or polycycloalkylidene group spiro-bound at the 3-carbon of said dioxetane; X is $CR_7R_8$, O, S, or N-R (wherein each of $R_7$, $R_8$, and R, independently, is H, alkyl, heteroalkyl, aryl, heteroaryl, cycloalkyl, cycloheteroalkyl, aralkyl, alkaryl, or an enzyme cleavable group); and each of $R_1$, $R_2$, $R_3$, $R_4$, $R_5$, and $R_6$, independently is H, an electron-withdrawing group, an electron-donating group, heteroaryl, or an enzyme cleavable group,

wherein the enzyme causes the dioxetane to decompose to form a luminescent substance having the formula

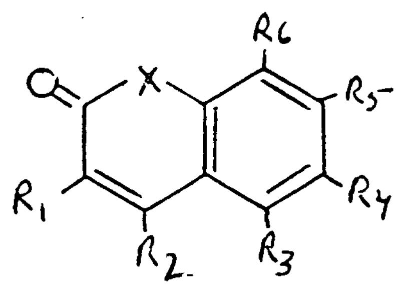

**14.** A method according to claim 13 wherein the group X is O, groups $R_1$-$R_3$ and $R_6$ are H, and groups $R_4$ and $R_5$, independently, are H or an enzyme cleavable group.

**15.** A method according to claim 13 or 14 wherein said dioxetane comprises an enzyme-cleavable group and said enzyme causes said dioxetane to decompose by cleaving said enzyme cleavable group from said dioxetane.

**16.** A method according to claim 15 wherein said enzyme cleavable group comprises phosphate and said enzyme comprises phosphatase.

**17.** A method according to claim 13 or 14 wherein said enzyme is an oxido-reductase and causes the dioxetane to decompose by cleaving the O-O bond of the 4-membered ring portion of said dioxetane.

**18.** A method according to claim 13 or 14 wherein said enzyme comprises a peroxidase.

**19.** A method according to claim 13 or 14 wherein said enzyme is horseradish peroxidase.

**20.** A method according to claim 13 or 14 wherein said enzyme is microperoxidase.

**21.** A method according to any of claims 13 to 20 wherein said specific binding pair comprises an antigen and an antibody.

**22.** A method according to any of claims 13 to 20 wherein said specific binding pair comprises a nucleic acid and a probe capable of binding to all or a portion of said nucleic acid.

**23.** A method of detecting an enzyme in a sample which method comprises the steps of
(a) providing a dioxetane having the formula

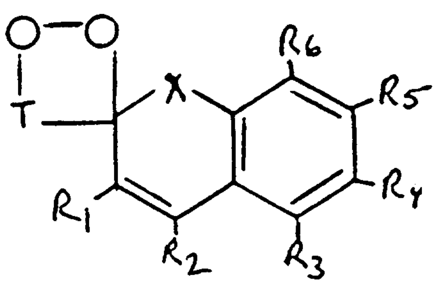

wherein T is a substituted or unsubstituted aryl, polyaryl, cycloalkylidene or polycycloalkylidene group spiro-bound at the 3-carbon of said dioxetane, X is $CR_7R_8$, O, S, or N-R (wherein each of $R_7$, $R_8$ and R, independently, is H, alkyl, heteroalkyl, aryl, heteroaryl, cycloalkyl, cycloheteroalkyl, aralkyl, alkaryl, or an enzyme cleavable group), and each of $R_1$, $R_2$, $R_3$, $R_4$, $R_5$, and $R_6$, independently, is H, an electron-withdrawing group, an electron-donating group, heteroaryl, or an enzyme cleavable group,
(b) contacting said dioxetane with said sample containing said enzyme,
whereupon said enzyme causes said dioxetane to decompose to form a luminescent substance having the formula

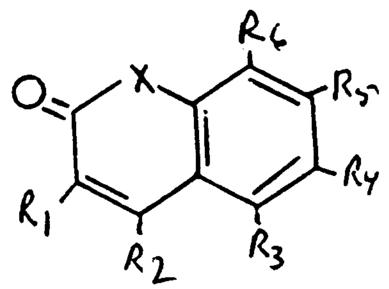

;

and

(c) detecting said luminescent substance as an indication of the presence of said enzyme.

**24.** A method according to claim 23 wherein said group X is O, groups $R_1$-$R_3$ and $R_6$ are H, and groups $R_4$ and $R_5$, independently, are H or an enzyme cleavable group.

**25.** A method according to claim 23 or 24 wherein said dioxetane comprises an enzyme-cleavable group and said enzyme causes said dioxetane to decompose by cleaving said enzyme cleavable group from said dioxetane.

**26.** A method according to claim 25 wherein said enzyme cleavable group comprises phosphate and said enzyme comprises phosphatase.

**27.** A method according to claim 23 or 24 wherein said enzyme is an oxido-reductase and causes said dioxetane to decompose by cleaving the O-O bond of the 4-membered ring portion of said dioxetane.

**28.** A method according to claim 23 or 24 wherein said enzyme comprises a peroxidase.

**29.** A method according to claim 23 or 24 wherein said enzyme is horseradish peroxidase.

**30.** A method according to claim 23 or 24 wherein said enzyme is microperoxidase.

**31.** A kit for detecting a first substance in a sample, the kit comprising

a dioxetane having the formula

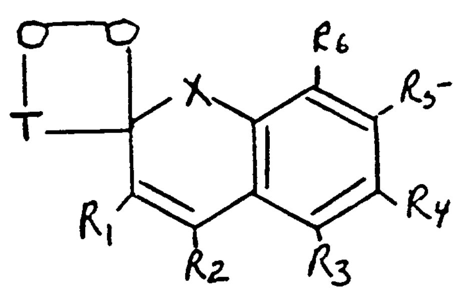

wherein T is a substituted or unsubstituted aryl, polyaryl, cycloalkylidene, or polycycloalkylidene group spiro-bound at the 3-carbon of said dioxetane, X is $CR_7R_8$, O, S, or N-R (wherein each of $R_7$, $R_8$, and R, independently, is H, alkyl, heteroalkyl, aryl, heteroaryl, cycloalkyl, cycloheteroalkyl, aralkyl, alkaryl, or an enzyme cleavable group), and each of $R_1$, $R_2$, $R_3$, $R_4$, $R_5$, and $R_6$, independently, is H, an electron-withdrawing group, an electron-donating group, heteroaryl, or an enzyme cleavable group; and

an enzyme capable of causing said dioxetane to decompose to form a fluorescent substance

having the formula

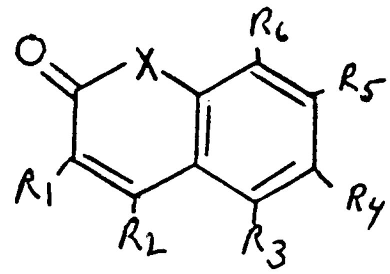

**Patentansprüche**

1. Dioxetan mit der Formel

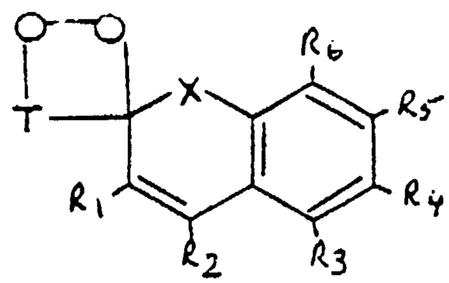

worin T eine substituierte oder unsubstituierte Aryl-, Polyaryl-, Cycloalkyliden- oder Polycycloalkylidengruppe ist, die an den 3-Kohlenstoff des Dioxetans spirogebunden ist; X $CR_7R_8$, O, S oder N-R ist (worin jeweils $R_7$, $R_8$ und R unabhängig voneinander H, Alkyl, Heteroalkyl, Aryl, Heteroaryl, Cycloalkyl, Cycloheteroalkyl, Aralkyl, Alkaryl oder eine Enzym-spaltbare Gruppe ist): und jeweils $R_1$, $R_2$, $R_3$, $R_4$, $R_5$ und $R_6$ unabhängig voneinander H, eine Elektronentziehende Gruppe, eine Elektron-spendende Gruppe, Heteroaryl oder eine Enzym-spaltbare Gruppe ist,
wobei das Dioxetan zur Zerlegung fähig ist, wodurch ein Leuchtstoff gebildet wird mit der Formel

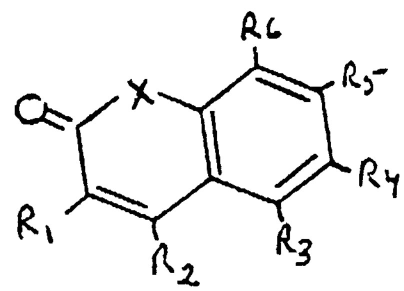

2. Dioxetan nach Anspruch 1, worin mindestens eine der Gruppen, T, $R_1$, $R_2$, $R_3$, $R_4$, $R_5$ und $R_6$, unabhängig voneinander eine Enzym-spaltbare Gruppe umfaßt.

3. Dioxetan nach Anspruch 2, worin die Enzym-spaltbare Gruppe Phosphat umfaßt.

4. Dioxetan nach Anspruch 1, worin die Gruppe X O ist, die Gruppen $R_1$-$R_3$ und $R_6$ H sind und die Gruppen $R_4$ und $R_5$ unabhängig voneinander H oder eine Enzym-spaltbare Gruppe sind.

5. Dioxetan nach einem der vorangegangenen Ansprüche, worin die Gruppe T eine Polycycloalkylidengruppe ist.

6. Dioxetan nach Anspruch 5, worin die Gruppe T Adamantyliden ist.

7. Verbindung mit der Formel

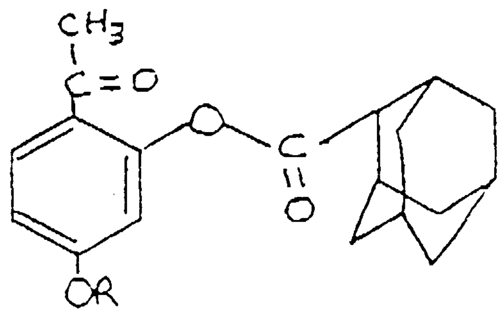

worin R H, eine $C_1$-$C_5$-Alkylgruppe, Acetyl oder $PO_4$ ist.

8. Verbindung mit der Formel

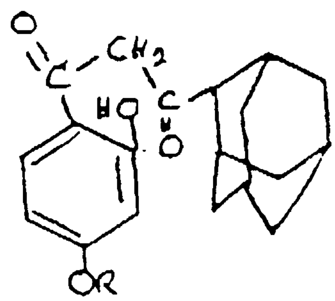

worin R H, eine $C_1$-$C_5$-Alkylgruppe, Acetyl oder $PO_4$ ist.

9. Verbindung mit der Formel

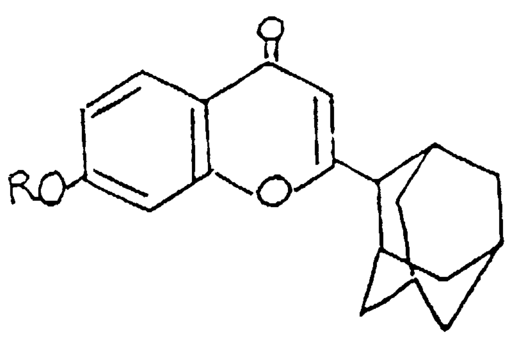

worin R H, eine $C_1$-$C_5$-Alkylgruppe, Acetyl oder $PO_4$ ist.

**10.** Verbindung mit der Formel

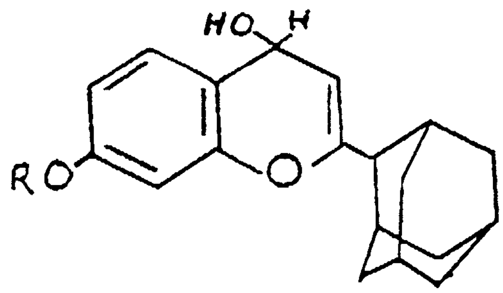

worin R H, eine $C_1$-$C_5$-Alkylgruppe, Acetyl oder $PO_4$ ist.

**11.** Verbindung mit der Formel

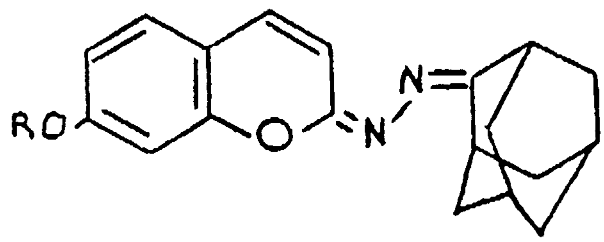

worin R H, eine $C_1$-$C_5$-Alkylgruppe, Acetyl oder $PO_4$ ist.

**12.** Verbindung mit der Formel

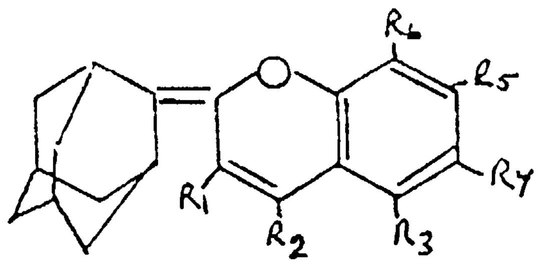

worin jeweils $R_1$, $R_2$, $R_3$, $R_4$, $R_5$ und $R_6$ unabhängig voneinander H, eine Elektron-entziehende Gruppe, eine Elektron-spendende Gruppe, Heteroaryl oder eine Enzymspaltbare Gruppe ist.

**13.** Assay-Verfahren, bei dem ein Bestandteil eines spezifischen Bindungspaares nachgewiesen wird mittels einer optisch nachweisbaren Reaktion, dadurch gekennzeichnet, daß die optisch nachweisbare Reaktion die Reaktion eines Dioxetans der Formel

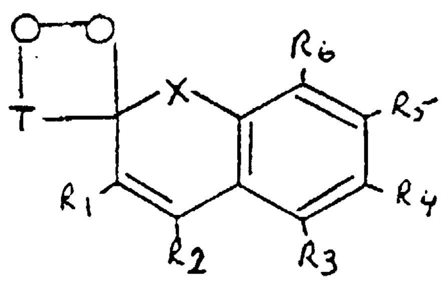

mit einem Enzym umfaßt, worin T eine substituierte oder unsubstituierte Aryl-, Polyaryl-, Cycloalkyliden- oder Polycycloalkylidengruppe ist, die an den 3-Kohlenstoff des Dioxetans spirogebunden ist; X $CR_7R_8$, O, S oder N-R ist (worin jeweils $R_7$, $R_8$ und R unabhängig voneinander H, Alkyl, Heteroalhyl, Aryl, Heteroaryl, Cycloalkyl, Cycloheteroalkyl, Aralkyl, Alkaryl oder eine Enzym-spaltbare Gruppe ist); und jeweils $R_1$, $R_2$, $R_3$, $R_4$, $R_5$ und $R_6$ unabhängig voneinander H, eine Elektron-entziehende Gruppe, eine Elektron-spendende Gruppe, Heteroaryl oder eine Enzym-spaltbare Gruppe ist,
worin das Enzym die Zerlegung der Dioxetans bewirkt, wodurch ein Leuchtstoff gebildet wird mit der Formel

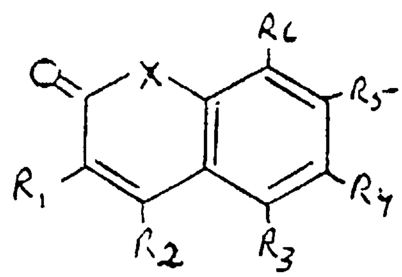

**14.** Verfahren nach Anspruch 13, worin die Gruppe X O ist, die Gruppen $R_1$-$R_3$ und $R_6$ H sind und die Gruppen $R_4$ und $R_5$ unabhängig voneinander H oder eine Enzym-spaltbare Gruppe sind.

**15.** Verfahren nach einem der Ansprüche 13 oder 14, worin das Dioxetan eine Enzym-spaltbare Gruppe umfaßt und das Enzym die Zerlegung des Dioxeatans bewirkt, indem die Enzym-spaltbare Gruppe vom Dioxetan abgespalten wird.

**16.** Verfahren nach Anspruch 15, worin die Enzym-spaltbare Gruppe Phosphat umfaßt und das Enzym Phosphatase umfaßt.

**17.** Verfahren nach einem der Ansprüche 13 oder 14, worin das Enzym eine Oxidoreduktase ist und die Zerlegung des Dioxetans bewirkt, indem die O-O-Bindung des 4-komponentigen Ringanteils des Dioxetans gespalten wird.

**18.** Verfahren nach Anspruch 13 oder 14, worin das Enzym eine Peroxidase umfaßt.

**19.** Verfahren nach Anspruch 13 oder 14, worin das Enzym Meerrettich-Peroxidase ist.

**20.** Verfahren nach Anspruch 13 oder 14, worin das Enzym Mikroperoxidase ist.

**21.** Verfahren nach einem der Ansprüche 13 bis 20, worin das spezifische Bindungspaar ein Antigen und einen Antikörper umfaßt.

**22.** Verfahren nach einem der Ansprüche 13 bis 20, worin das spezifische Bindungspaar eine Nukleinäure und eine Sonde umfaßt, die zum Binden an die gesamte oder einen Teil der Nukleinsäure fähig ist.

**23.** Verfahren zum Nachweis eines Enzyms in einer Probe, welches die folgenden Schritte umfaßt:

(a) Bereitstellen eines Dioxetans mit der Formel

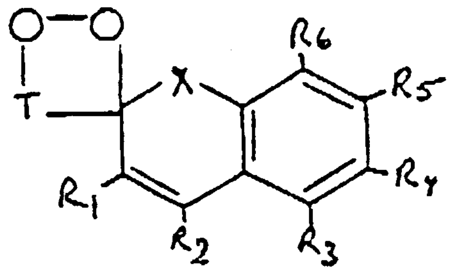

worin T eine substituierte oder unsubstituierte Aryl-, Polyaryl-, Cycloalkyliden- oder Polycycloalkylidengruppe ist, die an den 3-Kohlenstoff des Dioxetans spirogebunden ist; X $CR_7R_8$, O, S oder N-R ist (worin jeweils $R_7$, $R_8$ und R unabhängig voneinander H, Alkyl, Heteroalkyl, Aryl, Heteroaryl, Cycloalkyl, Cycloheteroalkyl, Aralkyl, Alkaryl oder eine Enzym-spaltbare Gruppe ist); und jeweils $R_1$, $R_2$, $R_3$, $R_4$, $R_5$ und $R_6$ unabhängig voneinander H, eine Elektron-entziehende Gruppe, eine Elektron-spendende Gruppe, Heteroaryl oder eine Enzym-spaltbare Gruppe ist.

(b) Zusammenbringen des Dioxetans mit der das Enzym enthaltenden Probe,
woraufhin das Enzym die Zerlegung des Dioxetans bewirkt, wodurch ein Leuchtstoff gebildet wird mit der Formel

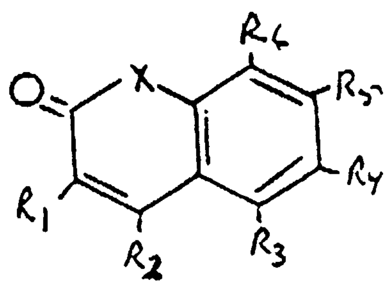

;

und

(c) Nachweisen des Leuchtstoffs als Anzeige für die Gegenwart des Enzyms.

**24.** Verfahren nach Anspruch 23, worin die Gruppe X O ist, die Gruppen $R_1$-$R_3$ und $R_6$ H sind und die Gruppen $R_4$ und $R_5$ unabhängig voneinander H oder eine Enzym-spaltbare Gruppe sind.

**25.** Verfahren nach Anspruch 23 oder 24. worin das Dioxetan eine Enzym-spaltbare Gruppe umfaßt und das Enzym die Zerlegung des Dioxetans bewirkt, indem die Enzym-spaltbare Gruppe vom Dioxetan abgespalten wird.

**26.** Verfahren nach Anspruch 25, worin die Enzym-spaltbare Gruppe Phosphat umfaßt und das Enzym Phosphatase umfaßt.

**27.** Verfahren nach Anspruch 23 oder 24, worin das Enzym eine Oxidoreduktase ist und die Zerlegung des Dioxetans bewirkt, indem die O-O-Bindung des 4-komponentigen Ringanteils des Dioxetans gespalten wird.

**28.** Verfahren nach Anspruch 23 oder 24, worin das Enzym eine Peroxidase umfaßt.

**29.** Verfahren nach Anspruch 23 oder 24, worin das Enzym Meerrettich-Peroxidase ist.

**30.** Verfahren nach Anspruch 23 oder 24, worin das Enzym Mikroperoxidase ist.

**31.** Bausatz zum Nachweis einer ersten Substanz in einer Probe, umfassend ein Dioxetan mit der Formel

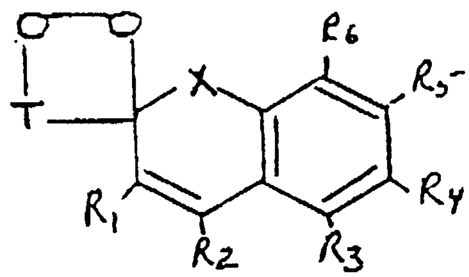

worin T eine substituierte oder unsubstituierte Aryl-, Polyaryl-, Cycloalkyliden- oder Polycycloalkylidengruppe ist, die an den 3-Kohlenstoff des Dioxetans spirogebunden ist; X $CR_7R_8$, O, S oder N-R ist (worin jeweils $R_7$, $R_8$ und R unabhängig voneinander H, Alkyl, Heteroalkyl, Aryl, Heteroaryl, Cycloalkyl, Cycloheteroalkyl, Aralkyl, Alkaryl oder eine Enzym-spaltbare Gruppe ist); und jeweils $R_1$, $R_2$, $R_3$, $R_4$, $R_5$ und $R_6$ unabhängig voneinander H, eine Elektron-entziehende Gruppe, eine Elektron-spendende Gruppe, Heteroaryl oder eine Enzym-spaltbare Gruppe ist; und

ein Enzym, das die Zerlegung des Dioxetans bewirken kann, wodurch ein Leuchtstoff gebildet wird mit der Formel

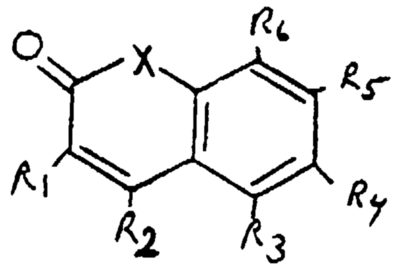

## Revendications

**1.** Dioxétanne de formule

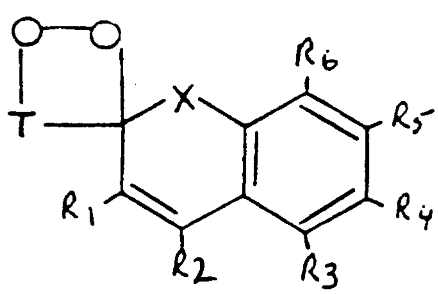

dans laquelle T est un groupe substitué ou non substitué aryle, polyaryle, cycloalkylidène ou polycycloalkylidène, lié par liaison spiro à l'atome de carbone en position 3 dudit dioxétanne ; X est $CR_7R_8$, O, S, ou N-R (où chacun de $R_7$, $R_8$ et R, indépendamment, est H ou un groupe alkyle, hétéroalkyle, aryle, hétéroaryle, cycloalkyle, cyclohétéroalkyle, aralkyle ou alkylarle ou un groupe clivable par une enzyme) ; et chacun de $R_1$, $R_2$, $R_3$, $R_4$, $R_5$ et $R_6$, indépendamment, est H, un groupe attracteur d'électrons, un groupe donneur d'électrons, un groupe hétéroaryle ou un groupe clivable par une enzyme,

ledit dioxétanne pouvant être décomposé et former une substance luminescente de formule

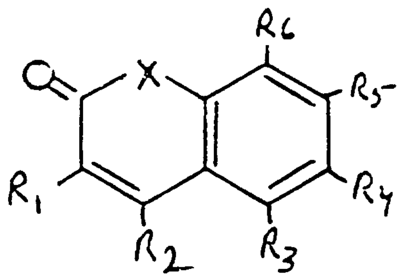

2. Dioxétanne selon la revendication 1, où au moins un desdits groupes T, $R_1$, $R_2$, $R_3$, $R_4$, $R_5$, et $R_6$, indépendamment, comprend un groupe clivable par une enzyme.

3. Dioxétanne selon la revendication 2, où ledit groupe clivable par une enzyme comprend un phosphate.

4. Dioxétanne selon la revendication 1, où le groupe X est O, les groupes $R_1$-$R_3$ et $R_6$ sont H et les groupes $R_4$ et $R_5$, indépendamment, sont H ou un groupe clivable par une enzyme.

5. Dioxétanne selon l'une quelconque des revendications précédentes, où le groupe T est un groupe polycycloalkylidène.

6. Dioxétanne selon la revendication 5, où le groupe T est un groupe adamantylidène.

7. Composé de formule

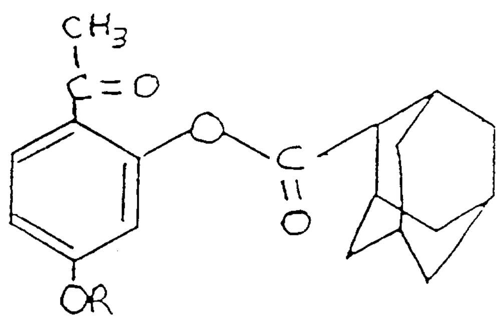

dans laquelle R est H, un groupe alkyle en $C_1$-$C_5$, un groupe acétyle ou $PO_4$.

8. Composé de formule

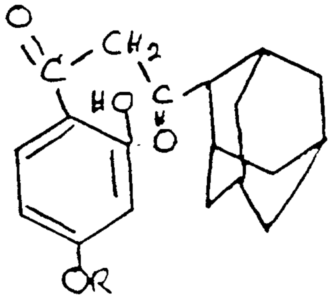

dans laquelle R est H, un groupe alkyle en $C_1$-$C_5$, un groupe acétyle ou $PO_4$.

**9.** Composé de formule

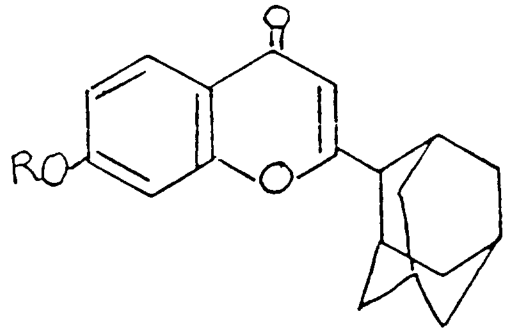

dans laquelle R est H, un groupe alkyle en $C_1$-$C_5$, un groupe acétyle ou $PO_4$.

**10.** Composé de formule

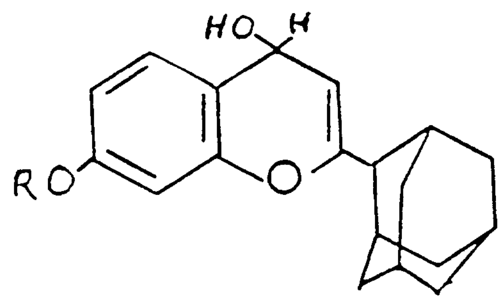

dans laquelle R est H, un groupe alkyle en $C_1$-$C_5$, un groupe acétyle ou $PO_4$.

**11.** Composé de formule

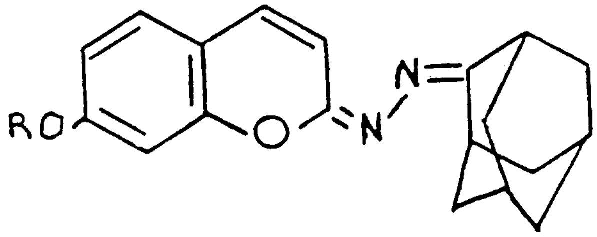

dans laquelle R est H, un groupe alkyle en $C_1$-$C_5$, un groupe acétyle ou $PO_4$.

**12.** Composé de formule

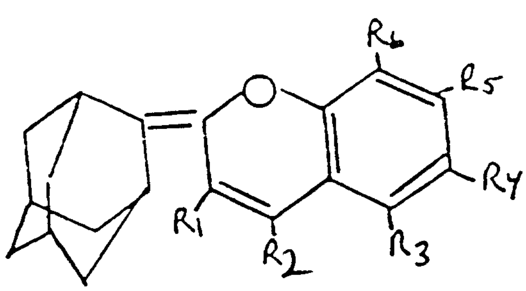

dans laquelle chacun de $R_1$, $R_2$, $R_3$, $R_4$, $R_5$ et $R_6$, indépendamment, est H, un groupe attracteur d'électrons, un groupe donneur d'électrons, un groupe hétéroaryle ou un groupe clivable par une enzyme.

**13.** Méthode d'analyse dans laquelle un membre d'un couple de liaison spécifique est détecté au moyen d'une réaction optiquement détectable, caractérisée en ce que la réaction optiquement détectable comprend la réaction, avec une enzyme, d'un dioxétanne de formule :

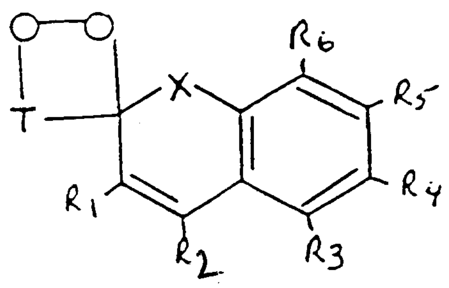

dans laquelle T est un groupe substitué ou non substitué aryle, polyaryle, cycloalkylidène ou polycycloalkylidène, lié par liaison spiro à l'atome de carbone en position 3 dudit dioxétanne ; X est $CR_7R_8$, O, S, ou N-R (où chacun de $R_7$, $R_8$ et R, indépendamment, est H ou un groupe alkyle, hétéroalkyle, aryle, hétéroaryle, cycloalkyle, cyclohétéroalkyle, aralkyle ou alkylaryle ou un groupe clivable par une enzyme) ; et chacun de $R_1$, $R_2$, $R_3$, $R_4$, $R_5$, et $R_6$, indépendamment, est H, un groupe attracteur d'électrons, un groupe donneur d'électrons, un groupe hétéroaryle ou un groupe clivable par une enzyme,

dans laquelle l'enzyme provoque la décomposition du dioxétanne et former une substance luminescente de formule

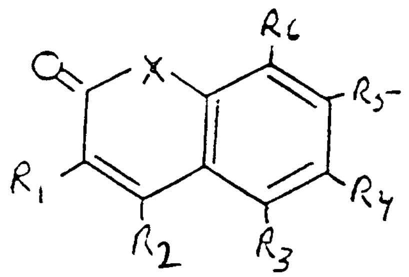

**14.** Méthode selon la revendication 13, dans laquelle le groupe X est O, les groupes $R_1$-$R_3$ et $R_6$ sont H et les groupes $R_4$ et $R_5$, indépendamment, sont H ou un groupe clivable par une enzyme.

**15.** Méthode selon la revendication 13 ou 14, dans laquelle ledit dioxétanne comprend un groupe clivable par une enzyme et ladite enzyme provoque la décomposition dudit dioxétanne par clivage dudit groupe clivable par une enzyme dudit dioxétanne.

**16.** Méthode selon la revendication 15, dans laquelle ledit groupe clivable par une enzyme comprend un phosphate et ladite enzyme comprend une phosphatase.

**17.** Méthode selon la revendication 13 ou 14, dans laquelle ladite enzyme est une oxydo-réductase et provoque la décomposition du dioxétanne par clivage de la liaison O-O de la portion de cycle à 4 chaînons dudit dioxétanne.

**18.** Méthode selon la revendication 13 ou 14, dans laquelle ladite enzyme comprend une peroxydase.

**19.** Méthode selon la revendication 13 ou 14, dans laquelle ladite enzyme est la peroxydase de raifort.

**20.** Méthode selon la revendication 13 ou 14, dans laquelle ladite enzyme est une microperoxydase.

**21.** Méthode selon l'une quelconque des revendications 13 à 20, dans laquelle ledit couple de liaison spécifique comprend un antigène et un anticorps.

**22.** Méthode selon l'une quelconque des revendications 13 à 20, dans laquelle ledit couple de liaison spécifique comprend un acide nucléique et une sonde capable de se lier à la totalité ou à une portion dudit acide nucléique.

**23.** Méthode de détection d'une enzyme dans un échantillon, comprenant les étapes consistant :
(a) à partir d'un dioxétanne de formule

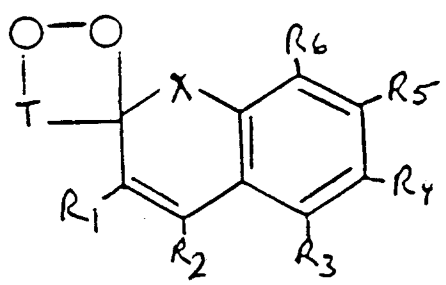

dans laquelle T est un groupe substitué ou non substitué aryle, polyaryle, cycloalkylidène ou polycycloalkylidène, lié par liaison spiro à l'atome de carbone en position 3 dudit dioxétanne ; X est $CR_7R_8$, O, S, ou N-R (où chacun de $R_7$, $R_8$ et R, indépendamment, est H ou un groupe alkyle, hétéroalkyle, aryle, hétéroaryle, cycloalkyle, cyclohétéroalkyle, aralkyle ou alylaryle ou un groupe clivable par une enzyme) ; et chacun de $R_1$, $R_2$, $R_3$, $R_4$, $R_5$ et $R_6$, indépendamment, est H, un groupe attracteur d'électrons, un groupe donneur d'électrons, un groupe hétéroaryle ou un groupe clivable par une enzyme, et
(b) à mettre en contact ledit dioxétanne avec ledit échantillon contenant ladite enzyme,
pour que ladite enzyme provoque la décomposition dudit dioxétanne et former une substance luminescente de formule

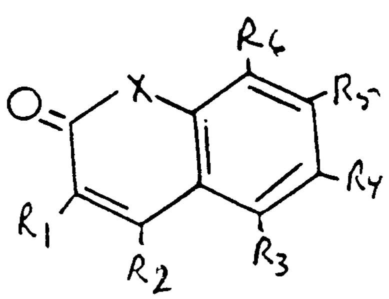

;

et
(c) détecter ladite substance luminescente en tant qu'indication de la présence de ladite enzyme.

**24.** Méthode selon la revendication 23, dans laquelle ledit groupe X est O, les groupes $R_1$-$R_3$ et $R_6$ sont H et les groupes $R_4$ et $R_5$, indépendamment, sont H ou un groupe clivable par une enzyme.

**25.** Méthode selon la revendication 23 ou 24, dans laquelle ledit dioxétanne comprend un groupe clivable par une enzyme et ladite enzyme provoque la décomposition dudit dioxétanne par clivage dudit groupe clivable par une enzyme dudit dioxétanne.

**26.** Méthode selon la revendication 25, dans laquelle ledit groupe clivable par une enzyme comprend un phosphate et ladite enzyme comprend une phosphatase.

**27.** Méthode selon la revendication 23 ou 24, dans laquelle ladite enzyme est une oxydo-réductase et provoque la décomposition dudit dioxétanne par clivage de la liaison O-O de la portion de cycle à 4 chaînons dudit dioxétanne.

**28.** Méthode selon la revendication 23 ou 24, dans laquelle ladite enzyme comprend une peroxydase.

**29.** Méthode selon la revendication 23 ou 24, dans laquelle ladite enzyme est la peroxydase de raifort.

**30.** Méthode selon la revendication 23 ou 24, dans laquelle ladite enzyme est une microperoxydase.

**31.** Nécessaire pour la détection d'une première substance dans un échantillon, le nécessaire comprenant un dioxétanne de formule

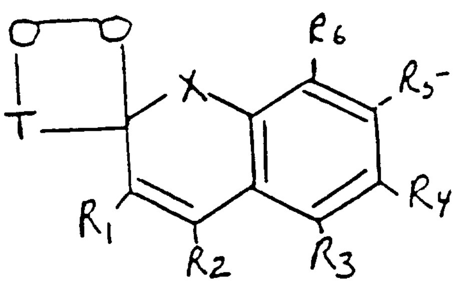

dans laquelle T est un groupe substitué ou non substitué aryle, polyaryle, cycloalkylidène ou polycycloalkylidène, lié par liaison spiro à l'atome de carbone en position 3 dudit dioxétanne ; X est $CR_7R_8$, O, S, ou N-R (où chacun de $R_7$, $R_8$ et R, indépendamment, est H ou un groupe alkyle, hétéroalkyle, aryle, hétéroaryle, cycloalkyle, cyclohétéroalkyle, aralkyle ou alkylaryle ou un groupe clivable par une enzyme), et chacun de $R_1$, $R_2$, $R_3$, $R_4$, $R_5$ et $R_6$, indépendamment, est H, un groupe attracteur d'électrons, un groupe donneur d'électrons, un groupe hétéroaryle ou un groupe clivable par une enzyme ; et

une enzyme capable de provoquer la décomposition dudit dioxétanne et former une substance fluorescente de formule